# EUROPEAN PATENT APPLICATION

(11) **EP 4 534 653 A1**
(43) Date of publication of application: **09.04.2025**
(21) Application number: 23915536.9
(22) Date of filing: 27.06.2023
(51) Int. Cl.: C12N 5/0783, C12N 5/078, A61K 35/17, A61P 35/00, A61P 37/02

(54) **METHOD FOR IN-VITRO INDUCTION AMPLIFICATION OF NK CELLS WITH HIGH PURITY AND HIGH CYTOTOXIC ACTIVITY**

(30) Priority: 10.01.2023 CN 202310035787
(71) Applicant: Shanghai Nk Cell Tech Co., Ltd., Shanghai 201318 (CN)
(72) Inventor: XIE, Siqi, Shanghai 201318 (CN); XU, Liang, Shanghai 201318 (CN); CHEN, Minhua, Shanghai 201318 (CN); XU, Huan, Shanghai 201318 (CN); FU, Yongling, Shanghai 201318 (CN)
(74) Representative: Dehns Germany Partnerschaft mbB
(86) International application number: PCT/CN2023/102675
(87) International publication number: WO 2024/148773

(57) **Abstract**

The present application relates to a method for *in vitro* induction and expansion of high-purity and high-cytotoxic NK cells. The method includes: inoculating isolated mononuclear cells in a pre-coated culture flask or a culture dish for culture treatment; and performing activation and induction culture in a culture medium containing monoclonal antibodies and cytokines, to finally obtain NK cells. The coating is performed in a buffer containing anti-CD 16 monoclonal antibody and IL-21.

## Description

### FIELD

The present disclosure belongs to the field of biomedicine. Specifically, the present disclosure relates to a preparation of high-purity and high-cytotoxic NK cells through *in vitro* induction and expansion. More specifically, the present disclosure relates to a method for culturing NK cells, NK cells, a pharmaceutical composition, and use of the pharmaceutical composition in the preparation of a medicament.

### BACKGROUND

In recent years, biological therapy has become the fifth major treatment mode following surgery, radiotherapy, chemotherapy, and endocrine therapy, and it has gradually attracted people's attention. Adoptive cellular immunotherapy (ACI), as one of the cellular biological therapies, involves the infusion of immune cells with anti-tumor activity into tumor patients to kill tumor cells directly or through stimulating body's immune response, thereby achieving the purpose of treating tumors. The adoptive immune cells currently used in clinical practice include DC-CIK cells, TIL cells, LAK cells, and NK cells. Among them, CIK cells, LAK cells, and A-NK cells are all anti-cancer systems with natural killer cells as the main body.

Natural killer cells, also referred to as NK cells, are mainly derived from bone marrow CD34+ lymphocytes and are distributed in the bone marrow, peripheral blood, and spleen. NK cells, accounting for 5% to 15% of peripheral blood lymphocytes, play an important role in tumor immunity and elimination of non-self cells. NK cells are the main members of natural immune defense and the first defensive line of body's defense system. The killing activity of NK cells is not restricted by major histocompatibility complex (MHC), and does not rely on antibodies. NK cells can be recognized without antigen pre-sensitization and kill tumor and virus-infected cells. NK cells directly exert cytotoxic effects on tumor cells through a perforin-granzyme pathway and a Fas-FasL pathway to kill tumor cells. At the same time, NK cells can also secrete a variety of cytokines and chemokines such as TNF-α, IFN-γ, and IL-I in the early stage of the disease. These cytokines are involved in anti-cancer reactions and the regulation of acquired immune responses. Therefore, NK cells are also a bridge connecting natural immunity and acquired immunity.

The safety and efficacy of NK cells in terms of anti-cancer effects have been confirmed. However, as NK cells only account for 5% to 15% of peripheral blood lymphocytes, it is pivotal to NK therapy to obtain NK cell products with high purity and high quality. In recent years, it has been discovered that relatively large-scale NK cell preparation may be performed through *in vitro* stimulation culture. Studies have indicated that cytokine such as IL-2 is the most common cytokine in *in vitro* expansion systems of NK cells. IL-2 can enhance the killing activity of NK cells and activate NK cells within a 24-hour reaction period, and thus promote the proliferation of NK cells and the production of cytokines. Cytokines such as IL-15, IL-18, and IL-12 also play a very important role in the activation and expansion of NK cells. IL-15 and IL-2, due to the synergistic effect thereof, can be used in combination for the *in vitro* expansion of NK cells. Meanwhile, these cytokines can also promote the directional differentiation of hematopoietic stem cells into NK cells by binding to the composite receptor γ chain expressed on the surface of NK cells, and play an important role in the development, differentiation, and long-term *in vitro* survival of NK cells.

At present, the large-scale preparation of NK cells faces the following challenges in terms of industrialization:
1. Conventional NK cell culture methods such as adding soluble recombinant growth factors, IL-2, IL-15, etc., have the disadvantages of limited expansion multiplicity and low killing activity.
2. The use of K562, as trophoblast cells (K562IL15-4-IBBligand) during expansion and culture of NK cells, does not meet GMP standards and raises safety concerns after irradiation of gamma rays. At the same time, although the use of allogeneic feeder cells from different sources (different individuals or tissues from the same species) has good expansion effects, most of the current schemes do not meet the national clinical use standards. In addition, the use of allogeneic feeder cells may lead to the spread of other diseases and bring about medical ethical issues.
   In addition, these methods cannot effectively exclude CD3+T lymphocytes, which still take a high proportion in the expansion system. Some researchers have tried to add anti-CD3 monoclonal antibodies into some culture systems with feeder cells, which can activate the feeder cells to the maximum extent, but bring about the risk of expansion and activation of residual CD3+ cells. A related risk of graft-versus-host disease (GVHD) also exists in the allogeneic feeding environment.
3. In order to improve the purity of NK cells, after obtaining mononuclear cells from peripheral blood, NK cell precursors or CD56+ are sorted and isolated using immunomagnetic beads to increase the proportion of NK cells. However, a cell sorting step is additionally performed, and the sorting and isolation method is expensive, which greatly increases the overall cost of cell culture. Moreover, the sorting process may also cause loss of NK cells, thereby reducing the number of cells and lowering the expansion efficiency of cells, which is unfavorable to large-scale culture applications.

Therefore, it is urgent to develop a preparation method for culturing high-purity and high-cytotoxic NK cells.

### SUMMARY

The present disclosure aims to solve one of the technical problems in the related art at least to a certain extent. To this end, the present disclosure provides a method for culturing NK cells. High-purity and high-cytotoxic NK cells are obtained by inoculating isolated mononuclear cells in a pre-coated culture flask or culture dish for culture treatment and performing induction and expansion treatment.

The present disclosure is based on the inventors' following findings:
Studies have shown that cytokines, as the most common cytokines in *in vitro* expansion systems of NK cells, can increase the killing activity of NK cells, activate NK cells, and promote the proliferation of NK cells and the production of cytokines in a short period of time. However, the large-scale preparation of NK cells has become a difficult subject in the industry due to defects such as limited expansion multiplicity, insufficient killing activity, graft-versus-host disease, and the possibility that allogeneic feeder cells may cause the spread of other diseases.

To this end, the present disclosure provides a method for culturing NK cells. By changing the culture conditions to culture NK cells on a large scale, NK cells with high purity, high cytotoxic activity and low graft-versus-host disease (GVHD) risk can be produced and do not have the problem that allogeneic feeder cells may cause the spread of other diseases.

Therefore, a first aspect of the present disclosure provides a method for culturing NK cells. According to an embodiment of the present disclosure, the method includes: inoculating isolated mononuclear cells (PBMCs) in a culture flask or a culture dish for culture treatment, the culture flask or the culture dish being subjected to pre-coating performed in a buffer containing anti-CD16 monoclonal antibody and IL-21. According to an embodiment of the present disclosure, the coating solution contains CD16, which can trigger an antibody-dependent cell-mediated cytotoxicity (ADCC) effect mediated by NK cells, causing the NK cells to release different cytotoxic molecules, thereby leading to the death of target cells. In addition, the inventors have found that the addition of IL-21 into the coating solution can regulate the proliferation and apoptosis ofB cells, promote the production and isotype switching of immunoglobulins, and enhance the cytotoxicity of CD8+T cells, natural killer cells, and NKT cells without causing apoptosis caused by activation. A simple way for enhancing the activity of ADCC is to stimulate NK cells with proinflammatory cytokines. The combination of anti-CD16 monoclonal antibodies and IL-21 can significantly enhance the activation of NK cells and the activity of ADCC.

The method for culturing NK cells adopted in the present disclosure has the characteristics of simple operation, fast expansion speed, high cell activity, high cell purity, high safety, and low cost, without causing medical ethics and GVHD risk issues, and it is suitable for large-scale preparation of NK cells and clinical application.

According to an embodiment of the present disclosure, the above-mentioned method for culturing NK cells may further include at least one of the following additional technical features.

According to an embodiment of the present disclosure, the buffer is selected from PBS buffer.

According to an embodiment of the present disclosure, a concentration of the anti-CD16 monoclonal antibody in the PBS buffer ranges from 1 mg/ml to 5 mg/ml, and preferably, 2 mg/ml. During the concentration optimization process, the inventors have found that, among the gradient concentrations ranging from 0.5 mg/ml to 5 mg/ml of CD16, the concentration of ≥2 mg/ml exhibited better cell proliferation ability and higher purity of NK cells. Therefore, preferably, the concentration of CD16 is 2 mg/ml.

According to an embodiment of the present disclosure, a concentration of the IL-21 in the PBS buffer ranges from 20 ng/ml to 100 ng/ml, and preferably, 50 ng/ml.

According to an embodiment of the present disclosure, the mononuclear cells are derived from peripheral blood.

According to an embodiment of the present disclosure, the culture treatment includes an activation treatment and an induction treatment.

According to an embodiment of the present disclosure, the activation treatment is performed in a first culture medium containing IL-2.

According to an embodiment of the present disclosure, a concentration of the IL-2 in the first culture medium ranges from 3.5 ng/ml to 6.5 ng/ml, and preferably, 5 ng/ml.

According to an embodiment of the present disclosure, the induction treatment is performed in a second culture medium containing anti-CD3 monoclonal antibody and interleukin.

According to an embodiment of the present disclosure, the interleukin includes at least one selected from IL-2, IL-12, and IL-15.

According to an embodiment of the present disclosure, the interleukin is IL-2. IL-2 is widely used to promote the activation and proliferation of T cells and NK cells. IL-2 can stimulate the proliferation of NK cells, increase cytotoxicity, and stimulate NK cells to secrete a variety of cytokines.

According to an embodiment of the present disclosure, a concentration of the anti-CD3 monoclonal antibody in the second culture medium ranges from 0.5 ng/ml to 5 ng/ml, and preferably, 1 ng/ml. The inventors found that the combined use of IL-2 and anti-CD3 monoclonal antibody can promote the expansion of NK cells derived from PBMCs. In addition, monoclonal antibodies against CD3 molecules can stimulate or block the transduction of activation signal of T cells, eliminating effector T cells or inducing the production of regulatory T cells. Through the experimental verification of concentration gradients ranging from 0.5 ng/ml to 5 ng/ml, it has been found that at least 1 ng/ml of CD3 monoclonal antibody may achieve the higher expansion effect of NK cells.

According to an embodiment of the present disclosure, a concentration of the IL-2 in the second culture medium ranges from 3.5 ng/ml to 6.5 ng/ml, and preferably, 5 ng/ml. The inventors have found that IL-2 has a positive regulatory effect on the activation and differentiation of NK cells. Experimental verification indicated that IL-2 at a concentration of 3.5 ng/ml can well induce the differentiation and proliferation of NK cells. During the culture process, the purity of NK cells is detected by verifying the number of cells and by means of flow cytometry, and it has been comprehensively confirmed that a high proportion of NK cells can be obtained in presence of IL-2 at a concentration of 5 ng/ml.

According to an embodiment of the present disclosure, a basal culture medium of the first culture medium and a basal culture medium of the second culture medium are each independently selected from at least one of CTS AIM V serum-free medium, generic DMEM F12 serum-free medium, KBM581 serum-free medium, X-vivo15, GT-551, and TBD.

According to an embodiment of the present disclosure, the basal culture media of the first culture medium and the second culture medium are each the KBM581 serum-free culture medium. By screening the aforementioned culture media, the inventor have ultimately found that the KBM581 serum-free culture medium had advantages in terms of expansion numbers of cells and the purity of NK cells.

A second aspect of the present disclosure provides NK cells. According to an embodiment of the present disclosure, the NK cells are prepared by the method according to the first aspect of the present disclosure.

The NK cells prepared according to the embodiments of the present disclosure have low cost, high cell activity, high cell purity, and good killing activity for tumors, and thus they can be prepared and applied on a large scale in clinical practice, without causing the occurrence of other diseases.

A third aspect of the present disclosure provides a pharmaceutical composition. According to an embodiment of the present disclosure, the pharmaceutical composition includes the NK cells according to the second aspect of the present disclosure.

According to an embodiment of the present disclosure, the pharmaceutical composition further includes a pharmaceutically acceptable excipient.

As mentioned above, the aforementioned NK cells have the advantages such as high cell activity, high cytotoxicity, good biocompatibility, strong delivery ability, and good immune activation effect. Delivery of the aforementioned NK cells into the body is beneficial for the NK cells to play a killing role on tumor cells.

A fourth aspect of the present disclosure provides use of a pharmaceutical composition in the preparation of a medicament. According to an embodiment of the present disclosure, the medicament is used for immunotherapy of a solid tumor or a hematological tumor.

According to an embodiment of the present disclosure, the solid tumor includes a tangible tumor occurring in an internal organ, including at least one of pancreatic cancer, ovarian cancer, mesothelioma, liver cancer, cholangiocarcinoma, gastric cancer, colorectal cancer, esophageal cancer, lung cancer, head and neck cancer, cervical cancer, brain glioma, renal cancer, breast cancer, prostate cancer, or melanoma.

According to an embodiment of the present disclosure, the hematological tumor includes at least one of acute myeloid leukemia, acute lymphoblastic leukemia, B-cell lymphoma, T-cell lymphoma, Hodgkin's lymphoma, non-Hodgkin's lymphoma, or multiple myeloma in blood cells and hematopoietic system.

A fifth aspect of the present disclosure provides a method for treating or preventing a solid tumor or a hematological tumor. According to an embodiment of the present disclosure, the method includes: administering at least one of the NK cells according to the second aspect or the pharmaceutical composition according to the third aspect to a subject. According to some specific embodiments of the present disclosure, a suitable dose of NK cells or pharmaceutical compositions is administered to a subject to inhibit the proliferation of solid tumor cells and/or hematological tumor cells.

According to an embodiment of the present disclosure, the above-mentioned method further includes at least one of the following technical features:
According to an embodiment of the present disclosure, the solid tumor includes a tangible tumor occurring in an internal organ, including at least one of pancreatic cancer, ovarian cancer, mesothelioma, liver cancer, cholangiocarcinoma, gastric cancer, colorectal cancer, esophageal cancer, lung cancer, head and neck cancer, cervical cancer, brain glioma, renal cancer, breast cancer, prostate cancer, or melanoma.

According to an embodiment of the present disclosure, the hematological tumor includes at least one of acute myeloid leukemia, acute lymphoblastic leukemia, B-cell lymphoma, T-cell lymphoma, Hodgkin's lymphoma, non-Hodgkin's lymphoma, or multiple myeloma in blood cells and hematopoietic system.

A sixth aspect of the present disclosure provides use of the NK cells according to the second aspect or the pharmaceutical composition according to the third aspect in the treatment or prevention of a solid tumor or a hematological tumor. According to some specific embodiments of the present disclosure, using the above-mentioned products, different treatment methods may be selected for different types of solid tumors or hematological tumors, including any one or a combination of the NK cells or pharmaceutical compositions, to improve the specificity and effectiveness of the treatment of solid tumors and/or hematological tumors.

According to an embodiment of the present disclosure, the above-mentioned method further includes at least one of the following technical features.

According to an embodiment of the present disclosure, the solid tumor includes a tangible tumor occurring in an internal organ, including at least one of pancreatic cancer, ovarian cancer, mesothelioma, liver cancer, cholangiocarcinoma, gastric cancer, colorectal cancer, esophageal cancer, lung cancer, head and neck cancer, cervical cancer, brain glioma, renal cancer, breast cancer, prostate cancer, or melanoma.

According to an embodiment of the present disclosure, the hematological tumor includes at least one of acute myeloid leukemia, acute lymphoblastic leukemia, B-cell lymphoma, T-cell lymphoma, Hodgkin's lymphoma, non-Hodgkin's lymphoma, or multiple myeloma in blood cells and hematopoietic system.

Additional aspects and advantages of the present disclosure will be provided in part in the following description, or will become apparent in part from the following description, or can be learned from practicing of the present disclosure.

### BRIEF DESCRIPTION OF THE DRAWINGS

The foregoing and/or additional aspects and advantages of the present disclosure will become apparent and more readily appreciated from the following descriptions of the embodiments made with reference to the accompanying drawings.
FIG. 1 is graphs showing detection results of proportions of NK cells in PBMCs before and after expansion by means of flow cytometer according to Example 1 of the present disclosure, the horizontal coordinate representing CD3, the vertical coordinate representing CD56, and CD3-CD56+ representing NK cells, the NK cells being expanded from 11.67% and 11.88% to 95.9% and 92.2%, respectively;
FIG. 2 is graphs of cell growth curves of NK/CD56+ cells with purity change during the expansion process according to Example 1 of the present disclosure;
FIG. 3 is expression graphs of NK cell surface activation receptors after expansion according to Example 1 of the present disclosure;
FIG. 4 is graphs showing killing results of NK cells after expansion against solid tumors, including ovarian cancer HO8910 cell line or melanoma A375 cell line, according to Example 2 of the present disclosure; and
FIG. 5 is graphs showing killing results of NK cells after expansion against hematological tumors, including leukemia K562 cell line and MOLT4 cell line, according to Example 2 of the present disclosure.

### DETAILED DESCRIPTION

Embodiments of the present disclosure will be described in detail below with reference to examples thereof as illustrated in the accompanying drawings, throughout which the same or similar elements or the elements having same or similar functions are denoted by same or similar reference numerals. The embodiments described below with reference to the drawings are illustrative only and intended to explain, rather than limiting, the present disclosure.

In the process of describing the present disclosure, the relevant terms herein are explained and illustrated only for the convenience of understanding the solution, and they cannot be regarded as limitations on the protection solution of the present disclosure.

As used herein, the terms "include" or "including" are open expressions, that is, including the contents specified in the present disclosure but not excluding other contents.

As used herein, the term "optional", or "optionally" generally means that the event or condition described thereafter may or may not occur, and the description includes situations in which the event or condition occurs, as well as situations in which the event or condition does not occur.

### Method for culturing NK cells

In one aspect of the present disclosure, a method for culturing NK cells is provided. According to an embodiment of the present disclosure, the method includes: inoculating isolated mononuclear cells (PBMCs) in a culture flask for culture treatment, which the culture flask is pre-coated with 1 mg/ml to 5 mg/ml of anti-CD16 monoclonal antibody and 20 ng/ml to 100 ng/ml of IL-21; and then performing induction culture with 0.5 mg/ml to 5 mg/ml of anti-CD3 monoclonal antibody and 3.5 ng/ml to 6.5 ng/ml of cytokine (at least one of IL-2, IL-12, or IL-15), to obtain high-activity and high-purity NK cells. The inventors found that the coating of the culture flask or the culture dish with a selected first medium containing anti-CD16 monoclonal antibodies and IL-21 has a function of significantly activating NK cells and enhancing the ADCC effect.

As used herein, antibody-dependent cell-mediated cytotoxicity (ADCC) is an effective cytotoxic mechanism primarily mediated by natural killer (NK) cells. The ADCC effect mainly activates NK cells by binding to antibodies of Fc receptor (FcR). The most characteristic FcR on the NK cell membrane is FcγRIII (CD16). CD16 is mainly responsible for triggering ADCC mediated by NK cells. When recognizing IgG-conditioned targets through CD16a, NK cells can release different cytotoxic molecules, thereby inducing the death of target cells. Such a mechanism relies on the formation of an immunological synapse and the degranulation of lytic granules containing perforin and granzymes. In addition to degranulation, NK cells may also bind target death receptors (such as DR4, DR5, or Fas) to death receptor ligands thereof (such as FasL and TRAIL) to eliminate the target cells. The various biological effects of IL-21 are mainly reflected in directly regulating the proliferation and apoptosis of B cells, promoting the production and isotype switching of immunoglobulins, and intensifying the cytotoxicity of CD8+T cells, natural killer cells, and NKT cells without causing apoptosis due to the activation. A simple way to enhance the activity of ADCC is to stimulate NK cells with proinflammatory cytokines. The combination of anti-CD16 monoclonal antibody and IL-21 can significantly enhance the activation of NK cells and the activity of ADCC.

It should be specifically noted that in the present disclosure, the mononuclear cells (PBMCs) are collected from a subject's blood cells, which are collected from the peripheral blood, umbilical cord blood, bone marrow, and/or lymph nodes of the subject.

The present disclosure provides an efficient and stable method for *in vitro* expansion of NK cells, having the following advantages. For the first advantage, the expansion method is simple, and the PBMCs isolated from the peripheral blood are directly used for subsequent culture after being treated with a combination of antibodies and cytokines, without requiring an additional step of sorting NK cells with magnetic beads. The sorting of NK cells with magnetic beads may increase the cost of expansion of cells and reduce the number of NK cells obtained and the efficiency of expansion of NK cells. For the second advantage, the culture flask is coated with a combination of two antibodies and cytokines to activate the cells, reduce the miscellaneous cells in PBMCs, and screen out most of the T cells. In this way, NK cells become dominant cells, the expansion of NK cells is stimulated and induced, significantly improving the purity of NK cells. For the third advantage, a combination of CD16 monoclonal antibody and interleukin cytokine IL-21 is used for culturing, which can induce the activity of the differentiation and proliferation of NK cells. For the fourth advantage, during the culture process, the culture solution is supplemented according to the growth conditions of cells, and the culture system is expanded, avoiding the excessive growth concentration of cells. In this way, the expansion efficiency of cells can be improved and the activity of cells can be maintained.

In yet another aspect of the present disclosure, the present disclosure provides a pharmaceutical composition. According to an embodiment of the present disclosure, the pharmaceutical composition includes the NK cells prepared as described above.

According to an embodiment of the present disclosure, the pharmaceutical composition further includes a pharmaceutically acceptable excipient.

As used herein, the term "pharmaceutically acceptable" indicates that the pharmaceutical composition may be administered to a subject without producing adverse physiological reactions that prevent the administration of the pharmaceutical composition. For example, "pharmaceutically acceptable excipients" refer to excipients that are useful in the preparation of generally safe, non-toxic, and desirable pharmaceutical compositions. Preferably, examples of these excipients or diluents include, but are not limited to, water, saline, Ringer's solution, glucose, mannitol, dextrose, lactose, starch, magnesium stearate, cellulose, magnesium carbonate, 0.3% glycerol, hyaluronic acid, ethanol, polyalkylene glycols such as polypropylene glycol, triglycerides, and 5% human serum albumin. Liposomes and nonaqueous mediators, such as non-volatile oils, may also be used.

Reference will now be made in more detail to the embodiments of the present disclosure, examples of which are illustrated in the accompanying drawings. The embodiments described below with reference to the figures are exemplary and are intended to be illustrative of the present disclosure and are not to be construed as limiting the present disclosure.

### Example 1: Expansion method of NK cells

### Coating of culture flask

The culture flask coating solution (PBS buffer containing anti-CD16 monoclonal antibody and IL-21) used in the present example was prepared by adding the following antibodies or cytokines, and diluted to reach the preferred concentration of anti-CD16 monoclonal antibody (Anti-CD16 mAb, 1 mg/ml to 5 mg/ml, 2 mg/ml was used in the present example) and IL-21 (interleukin 21, 20 ng/ml to 100 ng/ml, 50 ng/ml was used in the present example).
1. 15 ml of PBS buffer (commercial PBS, pyrogen- and endotoxin-free) was taken and added into a 50 mL centrifuge tube;
2. Anti-human CD16 monoclonal antibody and human IL-21 dissolved by PBS buffer were added into the 50 mL centrifuge tube in step 1, and the mixture was mixed thoroughly (and carefully for avoiding blowing bubbles), to obtain the coating solution;
3. The coating solution prepared in step 2 was added to two sterile T-75 culture flasks equally, allowing the coating solution to cover the bottom surface of the culture flasks evenly;
4. The coated T-75 culture flasks in step 3 were placed in a 4°C refrigerator for overnight coating (about 12 to 16 hours) to obtain the "NK initial culture flasks" (it is recommended to prepare the initial culture flasks one day before use and use them within 12 to 36 hours after the preparation);
5. Before use, the NK initial culture flasks were taken out from the 4°C refrigerator and restored to room temperature (in 10 to 20 minutes). Before use, the coating solution was discarded using a pipette as clean as possible (note: there is no need to clean the initial culture flasks), and the pipette did not touch the bottom of the flasks to avoid affecting the coating effect. T-75 culture flasks without coating solution were obtained and ready to be inoculated with cells.

### Isolation of PBMCs

In this example, the used NK cells were derived from human peripheral blood, and peripheral blood mononuclear cells (PBMCs) were isolated using the following steps and methods.
1. Preparation of GMP laboratory: Before entering the GMP laboratory, the air conditioner of the GMP laboratory was turned on half an hour in advance; the biological safety cabinet needed to be disinfected with ultraviolet light for 30 minutes before use; the table surface was wiped with 75% alcohol before use; 20 minutes after the turning-on of the biological safety cabinet, it was confirmed that the biological safety cabinet operated normally.
2. The peripheral blood of the donor was centrifuged at a speed of 800 g and at room temperature for 10 minutes, to isolate the cells and plasma, the centrifuge speed was 800 g, the and then speed was reduced to obtain layered blood cells and plasma.
3. The plasma in step 2 was pipetted using a sterile pipette in the biological safety cabinet, placed in another sterile centrifuge tube (50 ml), inactivated in a 56°C water bath for 30 minutes, placed at 4°C for 15 minutes, and then centrifuged at a speed of 800 g for 10 minutes. The upper layer of plasma was transferred to a new sterile centrifuge tube (50 ml), and stored at 4°C for later use. 0.9% saline for injection was added to the remaining blood cell sediment until the total volume was 1 to 1.2 times the volume of the whole blood, and inverted to mix. 15 ml to 20 ml of the blood cell suspension was taken and slowly injected into a 50ml centrifuge tube containing 15 ml of lymphocyte isolation solution, centrifuged at room temperature, 700 g×20 minutes (set values of rise and fall of the speed of centrifugation were 2 and 1, respectively), to obtain the isolation solution.
4. The mononuclear cell layer (lymphocytes and mononuclear cells) was pipetted from the isolation solution obtained in step 3 and transferred to a new sterile centrifuge tube (50 ml), not more than 15 ml per tube.
5. The centrifuge tube containing mononuclear cells in step 4 was filled with 0.9% saline for injection to reach a volume of 45 ml to 50 ml, inverted to mix, centrifuged at room temperature, 700 g for 10 minutes (set values of rise and fall of the speed of centrifugation were 9 and 9, respectively), and washed once. The supernatant was discarded, resuspended with 45 ml to 50 ml of 0.9% saline for injection, inverted to mix, centrifuged at room temperature, 500 g × 10 minutes (set values of rise and fall of the speed of centrifugation were 9 and 9, respectively), and washed once again. The supernatant was discarded. The prepared peripheral blood mononuclear cells were obtained.

### Initial culture of NK cells

According to an embodiment of the present disclosure, the initial culture of NK cells was performed using a first culture medium (KBM581 culture medium containing IL-2).
1. Day 0 of the culture: The prepared mononuclear cells were resuspended in KBM581 medium containing 5% autologous plasma (Corning Lymphocyte Serum-Free Medium KBM581, catalog number #88-581-CM, 1000 mL), and the density of cells was adjusted to 1.3×10⁶/ml. The cells were transferred to the prepared "NK initial culture flasks" (divided equally into 2 flasks, 40 to 45 ml/flask), and IL-2 was added to a final concentration of 5 ng/ml.
2. The cells were transferred to a 37°C incubator with saturated humidity and 5.0% CO₂ for continued culture.

### Induction (expansion) culture of NK cells

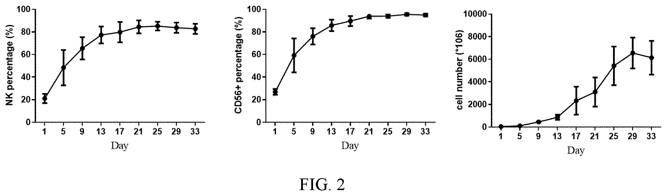

In the present disclosure, the induction and expansion culture of cells used NK expansion culture medium (the second culture medium), which was KBM581 serum-free culture medium supplemented with Anti-CD3mAb and IL-2.
1. Culture to the third day: IL-2 was added to a final concentration of 5 ng/ml, and the cells were placed in a 37°C incubator with saturated humidity and 5.0% CO₂ for continued culture.
2. Culture to the 5th day: the first transfer of culture of cells was performed.
   Fluid replenishment method: "NK expansion culture medium" (preparation method: 1 expansion factor was added into 1L KBM581 culture medium and mixed well) was added until the density of cells was 1.2×10⁶/ml, and IL-2 was added to 5 ng/ml and 2% autologous plasma was added according to the total volume.
3. Culture to the 7th day: The cells at the bottom of the flasks (only blow up the cells, were gently pipetted and then a small amount of cell suspension was taken out and mixed separately in the EP tube), and the cells were counted after mixing. When the density of cells did not exceed 1.5×10⁶/ml, transfer of culture was not necessary, but it was required to add IL-2 to 5 ng/ml and 2% autologous plasma according to the total volume. When the density of cells exceeded 1.5 × 10⁶/ml, transfer of culture was performed, "NK expansion culture medium" was added, the density of cells was adjusted to 1.4× 10⁶/ml, and the cells were continued to culture at 37°C and 5.0% CO₂.
4. Culture to the 9th to 33rd day: Generally, the transfer of culture was performed for the cells every two days. The transfer of culture was performed in the same way as step 3. "NK expansion medium" and IL-2 and autologous plasma were added (Note: When the density of cells was still not more than 1.5 ^{X} 10⁶/ml, the transfer of culture was not necessary and IL-2 was added to 5 ng/ml and 2% autologous plasma was added according to the total volume until the density of cells exceeded 1.5× 10⁶/ml. The density of cells was adjusted to 1.5×10⁶/ml during the transfer of culture . When the volume of the culture medium exceeded 60 ml, the culture medium was transferred to a T175 flask. When the volume of the culture medium exceeded 200 ml, the culture medium was transferred to a culture bag (TAKARA, 1.8 L specification).
5. During the initial culture of NK cells and the expansion culture of NK cells, the cell growth status was recorded. At different time periods of culture (1st, 5th, 9th, 13th, 17th, 21st, 25th, 29th, and 33rd days from the time of inoculation), the cell counting was performed and the NK cell growth curve was drawn (FIG. 2).

### Harvest of NK cells

1. If the number of cultured NK cells reached the required number, the cell suspension was taken for inspection and evaluation according to the specified items and methods of "finished product inspection standards" in the product quality control standards.
2. The cultured cells were transferred to a sterile centrifuge flask (250 ml) and collected by centrifugation at 850 g for 20 minutes; then the cells were washed twice with 0.9% saline for injection and centrifuged at 850 g for 8 minutes to obtain NK cells.
3. The NK cells were resuspended in 100 ml of 0.9% saline for injection. 5 ml of 20% human albumin was injected into the cell suspension using a 5 ml syringe to a final concentration of 1%. The mixture was mixed thoroughly and filtered through a 70 µm cell strainer to remove cell clumps. The cell counting was performed.

### Detection of purity and biological activity of NK cells

### 1) Experimental materials

Flow cytometry antibody: fluorescently labeled isotype control mouse monoclonal antibody: mouse IgG1-FITC (catalog number: 400108), mouse IgG1-Alexa Flour 647 (catalog number: 362514), mouse IgG1-Percpcy5.5 (catalog number: 400150), mouse IgG1-APC (catalog number: 400120), mouse IgG1-BV785 (catalog number: 400170), which were all purchased from Biolegend; surface molecule marker antibody: CD3-Percpcy5.5 (catalog number: 300430), CD56-BV785 (catalog number: 362550), FITC-CD3 (catalog number: 300406), CD56-Alexa Flour 647 (catalog number: 362514), NKG2D-APC (catalog number: 320808), Perforin-FITC (catalog number: 353310), and Granzyme B-Alexa Flour647 (catalog number: 515406), which were all purchased from Biolegend; DAPI (catalog number: 422801) purchased from Biolegend; and CFSE (catalog number: 51-9010817) purchased from BD Biosciences.

Blocking antibody: mouse serum, purchased from Future, catalog number F001008.

Flow cytometry tube: 5 ml transparent polystyrene or polypropylene round-bottom test tube with cap.

Monensin: purchased from Sigma, catalog number 22373-78-0.

Fixation/permeabilization Diluent: purchased from ebioscience, catalog number 4298341.

Fixation/permeabilization concentrate: purchased from ebioscience, catalog number 4311034.

Permeabilization buffer: purchased from ebioscience, catalog number 4314840.

DAPI (4',6-Diamidino-2-Phenylindole, Dilactate): purchased from Biolegend, catalog number 422801.

Flow cytometry wash buffer: PBS.

Relevant reagents from companies of the same grade and flow cytometry antibodies of the same clone number may also be used.

### 2) Main instruments and equipment

Centrifuge, super-clean bench, flow cytometer (such as FACS Calibur, Beckman CytoFlex, etc.), RTCA detection system.

### 3) Cell activity-related molecular detection methods

① Preparation of single-cell suspension of intracellular molecular labeled cells (monensin-induced): 4 × 10⁶ cultured cells (2 mL) were taken and placed in a 6-well plate, 10 µL of monensin (0.5 µg/µL) was added, cells were incubated at 37°C, 5% CO₂ constant temperature incubator for induction for 4 hours. After induction of intracellular molecular labeled cells with monensin, the cells were washed twice with 5 mL of 1×PBS and finally resuspended with 180 µL of 1×PBS into a single cell suspension.
② Preparation of single-cell suspension of surface molecular labeled cells: 9.0×10⁶ cultured NK cells were taken and placed in a 15 ml centrifuge tube, and the cells were collected by centrifugation at 400 g for 8 minutes. The cells were washed twice with 10 ml of 1× PBS, and finally resuspended with 0.81 ml of 1× PBS into a single-cell suspension.
③ Blocking: 90 µL of mouse serum was added to the surface molecular labeled cells obtained in step ②, mixing well, 20 µL of mouse serum was added to the intracellular molecular labeled cells obtained in step ①, mixing well, and allowing to stand at room temperature for 15 minutes to 30 minutes.
④ Antibody labeling: after blocking, the surface molecular labeled cells obtained in step ③ were divided into 9 flow tubes at 0.09 mL/tube. If multiple batches of cells were detected at the same time, the cells in tubes 1 to 8 may be labeled after mixing several batches of cells in equal amounts, and the number of cells in each tube was 0.8 to 1 × 10⁶ cells. Sample tube 1 was a surface molecular labeled, and the number of cells was 0.8 to 1 × 10⁶. Sample tube 2 was an intracellular molecular marker, and the number of cells was 3 × 10⁶ to 4 × 10⁶, which were added with the corresponding fluorescently labeled antibody, mixed well and the mixture stood for 30 minutes at 4°C while being protected from light (mixed well every 15 minutes during the process). Specific labeled methods were shown in Table 1.

**[Table 1] Sample tube for NK cell surface molecule detection and experimental operation**

| No. | Groups | Fluorescent labeled antibody | Amount |
|---|---|---|---|
| 1 | Blank control tube | / | / |
| 2 | Isotype control tube | mouse IgG1-FITC | 0.5 µl |
| | | mouse IgG1- Alexa Flour 647 | 0.5 µl |
| | | mouseIgG1-percp cy5.5 | 0.5 µl |
| | | mouse IgG1-APC | 0.5 µl |
| | | mouse IgG1-BV785 | 0.5 µl |
| 3 | Single-labeled control tube | CD3-FITC | 0.5 µl |
| 4 | | NKG2D-APC | 0.5 µl |
| 5 | | CD3- Percpcy5.5 | 0.5 µl |
| 6 | | CD56- BV785 | 0.5 µl |
| 7 | | CD56- Alexa Flour 647 | 0.5 µl |
| 8 | | DAPI | 5 µl |
| Sample tube 1 | Detection of surface molecular labeled cells | CD3-Percp cy5.5; | 0.5 µl |
| | | CD56- BV785 | 0.5 µl |
| | | NKG2D-APC | 0.5 µl |
| | | DAPI | 5 µl |
| Sample tube 2 | Detection of intracellular molecular labeled cells | CD3-Percp cy5.5; | 1 µl |
| | | CD56- BV785; | 1 µl |
| | | Perforin-FITC | 1 µl |
| | | Granzyme B-Alexa Fluor 647 | 1 µl |

⑤ Washing: 1 mL of 1×PBS was added to each sample tube 1 (surface molecule labeled cells), and the cells were harvested by centrifuging at 4°C, 400 g for 8 minutes. The cells were washed 2 times with 1 mL of 1×PBS. Finally, 200 µL of 1×PBS was added to each tube to resuspend the labeled cells, 5 µL of DAPI (50 µg/mL, 40x) was added to the experimental group, and the tube was transferred to the machine for detection. After labeling with external standard molecules CD3 and CD56, 5 µl of DAPI was added to sample tube 2 (intracellular molecule labeled cells), and labeled for 3 minutes. 1 ml of PBS was added to each tube and washed twice, and the supernatant was discarded (note: discard as much as possible). 300 µl of Fixation Buffer mix (Fixation/permeabilization concentrate: Fixation/permeabilization Diluent=1: 3) was added to the tube, and fixed in a 4°C refrigerator for 1 hour (mixed evenly every 30 minutes). After completion, 1 ml of Perm Wash Buffer (storage solution was 10× concentrated, diluted with double-distilled water to 1× for use) was added, centrifuged at 500 g and 4°C for 5 minutes, and the supernatant was discarded. Then, the cells were resuspended with 180 µl of Perm Wash Buffer, and 20 µl of mouse serum was added and blocked in a 4°C refrigerator for 20 minutes. After blocking, the cells were divided into two equal parts and labeled with antibodies. Specific labeled methods were shown in Table 2. The cells were labeled in the 4°C refrigerator for 1 hour under light protection (mixed once in the middle), and after the antibody labeling, 1ml of Perm Wash Buffer was added and centrifuged at 500 g and 4°C for 5 minutes. The cells were washed once again. Finally, the cells were resuspended with 200µl of Perm Wash Buffer and detected on the machine.
⑥ Detection: the flow cytometer was calibrated and adjusted according to the instructions for use. The blank control tube samples were used to adjust the forward and side scatter voltages. The isotype control samples were used to adjust the voltage of each fluorescence channel to keep its fluorescence within the negative range. The single-labeled control samples were used to adjust the fluorescence compensation of each channel. After the cell gates were delineated during the assay, 1 × 10⁴ cells were collected in each sample gate.

**[Table 2] Antibody-labeled intracellular molecular labeled cells**

| Sample | Labeling components | |
|---|---|---|
| Sample tube 2-1 | FITC-mouse IgG1.x | APC-mouse IgG1.κ |
| Sample tube 2-2 | FITC-perforin | APC-granzymeB |

### 4) Determination of validity of experimental results

The positive rate of fluorescent staining of blank control tube samples was <1%, and the absolute number of cells was less than 100. The positive rates of isotype controls of five different fluorescent-labeled antibodies were all <1%, and the absolute number of cells was less than 100 cells. The percentage of positive cells (positive cells%) = (number of positive cells = total number of counted cells 1×104) × 100%.

The experimental results meeting the above requirements were qualified. Otherwise, they were unqualified.

### Detection of expansion results

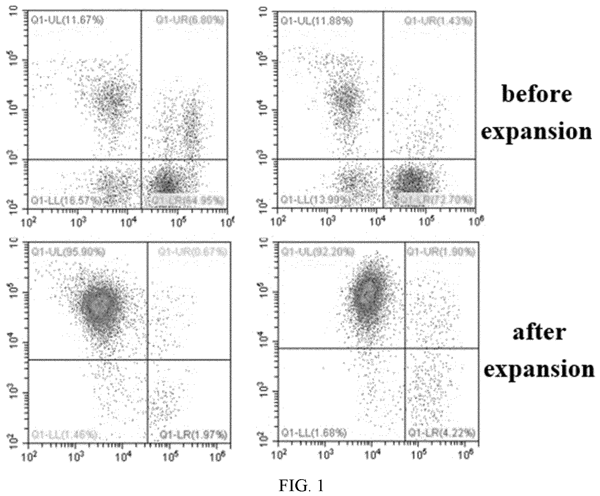

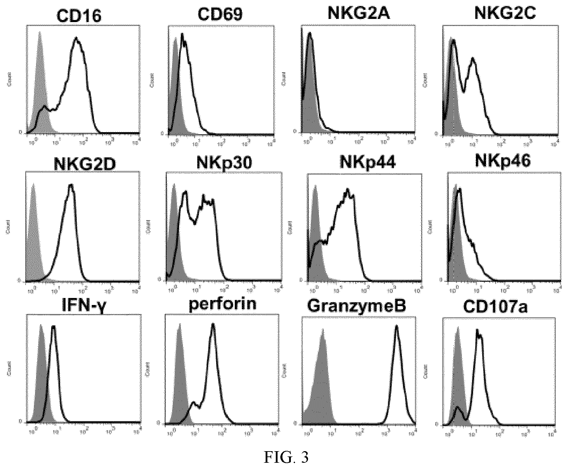

It can be seen from the detection results of flow cytometry of NK cells (FIG. 1) that the NK cells increased from 11.67% and 11.88% before expansion to 95.9% and 92.2% after expansion, and the NK cells obtained after expansion had a high purity. From the statistical results of five repeated experiments (N=5) (FIG. 2), it can be seen that: the cell growth efficiency was high, the ratio of NK% and CD56+% increased rapidly, the number of NK cells increased by 1000 times to 2000 times during the expansion cycle, and CD56+ may reach more than 90%. From the expression results of NK cell surface activation receptors after expansion (FIG. 3), it can be seen that the expression of NK cell surface activation receptors after expansion detected by flow cytometry was significantly enhanced compared with that before expansion.

### Example 2: Detection method of killing activity of cells

The real-time label-free cell analysis technology was used to detect the killing activity of NK cells against solid tumors (applicable to adherent cells. The solid tumors verified in this example include: ovarian cancer cell line HO8910 or melanoma A375 cell line).

### Preparation of target cell suspension (Day 1)

1. In a super-clean bench, the old culture medium was removed from the culture dish under sterile conditions.
2. Then, the culture dish was washed twice with 1×PBS and added with 1 mL of trypsin solution containing EDTA. The mixture was digested at room temperature for 2 to 6 minutes.
3. The trypsin solution was discarded, and an appropriate amount of culture medium was added to the culture dish. The cells were gently pipetted to obtain a cell suspension, which was centrifuged at 140 g for 5 minutes. After centrifugation, the cells were resuspended in 1 mL of complete culture medium. 20 µl of suspension was taken for cell counting, and a cell concentration of 1.6×10⁵ cells/ml required for the experiment was prepared

### RTCA DP system calibration and target cell culture

1. 50 µl of culture medium was added into the wells of E-Plate 16.
2. The E-Plate 16 was placed on RTCA Station.
3. The RTCA system automatically scanned ("Scan Plate") -> check whether the contact was good (Connection OK would be displayed on the "Message" page).
4. Start detecting the baseline (Background) -> confirm that the selected hole was in normal contact.
5. The E-Plate 16 was taken out, and 100 µl of the mixed target cell suspension prepared above was added to the wells of the E-Plate 16, allowing the number of cells in each well to be 1.6×10⁴ cells/well (Note: After adding the cells to E-Plate 16, there was no need to mix the cells and the original culture medium in the wells).
6. The E-Plate 16 was placed on the super-clean bench for 20 minutes to allow the cells to settle to the bottom of the plate.
7. The E-Plate 16 was placed on RTCA Station in the incubator.
8. After the system automatically scanning "Scan Plate", Step 2 was initiated to perform real-time dynamic detection of cell proliferation.
9. When the target cells have grown for about 12 hours and the cell index reached about 2.0, start preparing to add NK cells.

### Preparation of NK cells to be detected and killing experiment (Day 2)

1. The prepared NK cells were prepared at an appropriate density of cells according to the effective target ratio (ratio of effector cells to target cells).
2. The E-Plate 16 was taken out and placed in the super-clean bench.
3. 50 ul of NK cell suspension (or an equal volume of culture medium: blank control) was added into the culture plate at different effective target ratios (1:1, 2:1, 4:1, 8:1). In addition, control wells containing only target cells and only NK cells should be set up.
4. The E-Plate 16 detection plate was placed on the RTCA DP detection station for real-time monitoring to observe the real-time killing effect of NK cells on HO8910 cells.
5. Calculation method of results: (average cell index of control group - average cell index of experimental group)/average cell index of control group × 100%.

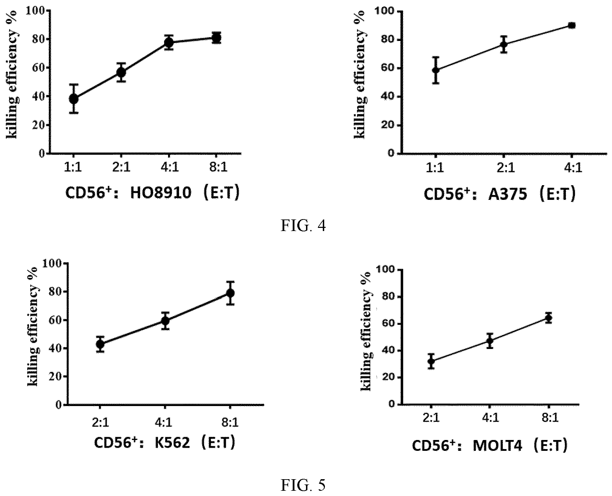

The results indicated that the expanded NK cells had a higher killing function on tumor cells. For HO8910 cells, a killing efficiency of about 80% can be achieved when the effective target ratio was 4:1 (FIG. 4). For A375 cells, a killing efficiency of about 90% can be achieved when the effective target ratio was 4:1 (FIG. 4). The killing of both tumor cells by NK cells showed a dose-effect relationship, and the killing efficiency was gradually enhanced with the increase of the effective target ratio.

**Detection of killing ability of NK cells against hematological tumors by means of flow cytometry (applicable to suspension cells, in this example, hematological tumors including leukemia K562 cell line and MOLT4 cell line were tested).**

### Preparation of target cell suspension:

1. Target cell (K562 cell) counting: the cells were suspended in 1640 culture medium containing 0.5% FBS (the culture medium used for target cells), counted, and the density of cells was adjusted to 1 × 10⁶/mL.
2. Fluorescent dye hydroxyfluorescein diacetate succinimidyl ester (CFSE) staining: CFSE (working concentration: 5 µM) was added to the cell suspension prepared in step 1, and the cells were pipetted and mixed well with 1 mL pipette, followed by vortex, incubating for 15 minutes in a 37°C incubator protected from light, and taking out every 5 minutes to vortex well to obtain the stained target cells.
3. Stop staining: the staining was stopped by adding 5 volumes of complete culture medium precooled at 4°C (the culture medium used for target cells) to the stained target cell solution in step 2 and incubated on ice for 5 minutes, followed by centrifuging at 140 g and 4°C for 5 minutes.
4. Washing target cells: complete culture medium precooled at 4°C (the culture medium used for target cells) was used to re-suspend the cells, and the cells were centrifuged at 140 g and 4°C for 5 min. The washing was repeated twice.
5. Counting: the target cells were resuspended and counted, and the cell density was adjusted to 2 × 10⁵/mL.
6. Plating: 100 µl of target cell suspension was added into each well of the 96-well round bottom plate, enabling the final cell number in each well to be 20,000 cells/well.
7. NK cells were prepared at a suitable density.
8. The E-Plate 16 was taken and placed on a super-clean bench.
9. 100 µl of NK cell suspension at different effective target ratios (5:1, 2.5:1, 1.25:1) was added to the culture plate. In addition, three groups of controls were provided, i.e., only target cells stained with CFSE only (for detecting natural mortality of target cells); only effector cells (for demonstrating that effector cells do not contain CFSE non-specific staining); and target cells + Tween-20 (as positive control for apoptosis of target cells);
10. Co-incubation, NK cells were added to each well in a pre-designed order. The effector cells and target cells were contacted thoroughly by centrifugation at 120 g and at room temperature for 2 minutes. The cells were returned for incubating in a 37°C incubator for 4 hours.
11. At the end of incubation, 1 µl of 7-AAD (7-aminoactinomycin D) was added, mixed well, incubated for 5 min protected from light, and detected on the machine.
12. Analysis of results: percentage of killing = [(death rate (%) of target cells in experimental group-death rate (%) of target cells)/(100%-natural death rate (%) of target cells)] × 100%).

The results showed that NK cells were co-incubated with tumor cell lines at different ratios of effector cells and target cells, and the death rate of target cells was detected after 4 hours. Since K562 cells are sensitive to the killing of NK cells, a killing efficiency of about 80% may be achieved when the effective target ratio was 8:1 (FIG. 5). For MOLT4 cells, a killing efficiency of about 60% may be achieved when the effective target ratio was 8:1 (FIG. 5). The killing of both tumor cells by NK cells showed a dose-effect relationship, and the killing efficiency was gradually enhanced with the increase of the effective target ratio.

Furthermore, the terms "first" and "second" are used for descriptive purposes only and are not to be construed as indicating or implying relative importance or implicitly indicating the number of technical features indicated. Thus, a feature defined as "first" or "second" may explicitly or implicitly include at least one such feature. In the description of the present disclosure, the meaning of "plurality" is at least two, e.g. two, three, etc. unless specifically and specifically limited otherwise.

In the specification, the description of the reference terms such as "one embodiment", "some embodiments", "example", "specific example", or "some example" means that the specific features, structures, materials, or characteristics described with reference to the embodiment or example are included in at least an embodiment or example of the present disclosure. In this specification, exemplary descriptions of the foregoing terms do not necessarily refer to the same embodiment or example. Moreover, the specific features, structures, materials, or characteristics described may be combined in any one or more embodiments or examples in a suitable manner. Furthermore, those skilled in the art may combine different embodiments or examples and features of different embodiments or examples described in this specification, unless they are contradictory to each other.

Although embodiments of the present disclosure are illustrated and described above, it can be understood that the above embodiments are illustrative and should not be construed as limitations of the present disclosure. Those skilled in the art can make changes, modifications, substitutions, and variations to the above embodiments within the scope of the present disclosure.

## Claims

1. A method for culturing NK cells, comprising:
inoculating isolated mononuclear cells in a culture flask or a culture dish for culture treatment, wherein the culture flask or the culture dish is subjected to pre-coating performed in a buffer containing anti-CD16 monoclonal antibody and IL-21.

2. The method according to claim 1, wherein the buffer is selected from PBS buffer.

3. The method according to claim 1 or 2, wherein a concentration of the anti-CD16 monoclonal antibody in the PBS buffer ranges from 1 mg/ml to 5 mg/ml.

4. The method according to claim 3, wherein the concentration of the anti-CD16 monoclonal antibody in the PBS buffer is 2 mg/ml.

5. The method according to claim 1 or 2, wherein a concentration of the IL-21 in the PBS buffer ranges from 20 ng/ml to 100 ng/ml.

6. The method according to claim 5, wherein the concentration of the IL-21 in the PBS buffer is 50 ng/ml.

7. The method according to claim 1, wherein the mononuclear cells are derived from peripheral blood.

8. The method according to claim 1, wherein the culture treatment comprises an activation treatment and an induction treatment.

9. The method according to claim 8, wherein the activation treatment is performed in a first culture medium containing IL-2.

10. The method according to claim 9, wherein a concentration of the IL-2 in the first culture medium ranges from 3.5 ng/ml to 6.5 ng/ml.

11. The method according to claim 10, wherein the concentration of the IL-2 in the first culture medium is 5 ng/ml.

12. The method according to claim 8, wherein the induction treatment is performed in a second culture medium containing anti-CD3 monoclonal antibody and interleukin.

13. The method according to claim 12, wherein the interleukin comprises at least one selected from IL-2, IL-12, and IL-15.

14. The method according to claim 13, wherein the interleukin is IL-2.

15. The method according to claim 12, wherein a concentration of the anti-CD3 monoclonal antibody in the second culture medium ranges from 0.5 ng/ml to 5 ng/ml.

16. The method according to claim 15, wherein the concentration of the anti-CD3 monoclonal antibody in the second culture medium is 1 ng/ml.

17. The method according to claim 12 or 14, wherein a concentration of the IL-2 in the second culture medium ranges from 3.5 ng/ml to 6.5 ng/ml.

18. The method according to claim 17, wherein the concentration of the IL-2 in the second culture medium is 5 ng/ml.

19. The method according to claim 9 or 12, wherein a basal culture medium of the first culture medium and a basal culture medium of the second culture medium are each independently selected from at least one of CTS AIM V serum-free medium, generic DMEM F12 serum-free medium, KBM581 serum-free medium, X-vivo15, GT-551, and TBD.

20. The method according to claim 19, wherein the basal culture media of the first culture medium and the basal culture media of the second culture medium are each the KBM581 serum-free culture medium.

21. NK cells, prepared by the method according to any one of claims 1 to 20.

22. A pharmaceutical composition, comprising the NK cells according to claim 21.

23. The pharmaceutical composition according to claim 22, further comprising a pharmaceutically acceptable excipient.

24. Use of the NK cells according to claim 21 or the pharmaceutical composition according to any one of claims 22 to 23 in the preparation of a medicament for immunotherapy of a solid tumor or a hematological tumor.

25. The use according to claim 24, wherein the solid tumor comprises a tangible tumor occurring in an internal organ, the tangible tumor comprising at least one of pancreatic cancer, ovarian cancer, mesothelioma, liver cancer, cholangiocarcinoma, gastric cancer, colorectal cancer, esophageal cancer, lung cancer, head and neck cancer, cervical cancer, brain glioma, renal cancer, breast cancer, prostate cancer, or melanoma.

26. The use according to claim 24, wherein the hematological tumor comprises at least one of acute myeloid leukemia, acute lymphoblastic leukemia, B-cell lymphoma, T-cell lymphoma, Hodgkin's lymphoma, non-Hodgkin's lymphoma or multiple myeloma in blood cells and hematopoietic system.

27. A method for treating or preventing a solid tumor or a hematological tumor, comprising:
administering at least one of the NK cells according to claim 21 or the pharmaceutical composition according to any one of claims 22 to 23 to a subject.

28. The method according to claim 27, wherein the solid tumor comprises a tangible tumor occurring in an internal organ, the tangible tumor comprising at least one of pancreatic cancer, ovarian cancer, mesothelioma, liver cancer, cholangiocarcinoma, gastric cancer, colorectal cancer, esophageal cancer, lung cancer, head and neck cancer, cervical cancer, brain glioma, renal cancer, breast cancer, prostate cancer, or melanoma.

29. The method according to claim 27, wherein the hematological tumor comprises at least one of acute myeloid leukemia, acute lymphoblastic leukemia, B-cell lymphoma, T-cell lymphoma, Hodgkin's lymphoma, non-Hodgkin's lymphoma or multiple myeloma in blood cells and hematopoietic system.

30. Use of the NK cells according to claim 21 or the pharmaceutical composition according to any one of claims 22 to 23 in the treatment or prevention of a solid tumor or a hematological tumor.

31. The use according to claim 30, wherein the solid tumor comprises a tangible tumor occurring in an internal organ, the tangible tumor comprising at least one of pancreatic cancer, ovarian cancer, mesothelioma, liver cancer, cholangiocarcinoma, gastric cancer, colorectal cancer, esophageal cancer, lung cancer, head and neck cancer, cervical cancer, brain glioma, renal cancer, breast cancer, prostate cancer, or melanoma.

32. The use according to claim 30, wherein the hematological tumor comprises at least one of acute myeloid leukemia, acute lymphoblastic leukemia, B-cell lymphoma, T-cell lymphoma, Hodgkin's lymphoma, non-Hodgkin's lymphoma or multiple myeloma in blood cells and hematopoietic system.
